# EUROPEAN PATENT SPECIFICATION

(11) **EP 3 556 351 B1**
(45) Date of publication and mention of the grant of the patent: **09.08.2023**
(21) Application number: 19386016.0
(22) Date of filing: 22.03.2019
(51) Int. Cl.: A61K 9/08, A61K 9/14, A61K 31/375, A61K 31/519, A61K 33/26, A61K 47/26

(54) **A PHARMACEUTICAL COMPOSITION FOR ENHANCING IRON LEVELS IN BLOOD, COMPRISING IRON (III) POLYMALTOSE AND ANOTHER ACTIVE SUBSTANCE, SUITABLE FOR IRON ABSORPTION, SELECTED FROM CALCIUM FOLINATE PENTAHYDRATE, VITAMIN C OR A COMBINATION OF BOTH**
PHARMAZEUTISCHE ZUSAMMENSETZUNG ZUR ERHÖHUNG DES EISENSPIEGELS IM BLUT MIT EISEN(III)-POLYMALTOSE UND EINEM ANDEREN ZUR EISENABSORPTION GEEIGNETEN WIRKSTOFF, AUSGEWÄHLT AUS CALCIUMFOLINATPENTAHYDRAT, VITAMIN C ODER EINER KOMBINATION AUS BEIDEN
COMPOSITION PHARMACEUTIQUE PERMETTANT D'AMÉLIORER LES NIVEAUX DE FER DANS LE SANG, COMPRENANT DU POLYMALTOSE DE FER (III) ET UNE AUTRE SUBSTANCE ACTIVE ADAPTÉE À L'ABSORPTION DU FER CHOISIE PARMI LE FOLINATE DE CALCIUM PENTAHYDRATÉ, LA VITAMINE C OU UNE COMBINAISON DES DEUX

(30) Priority: 16.04.2018 GR 20180100160
(43) Date of publication of application: 23.10.2019
(73) Proprietor: Tseti, Ioulia, 145 61 Kifissia Attica (GR)
(72) Inventor: Tseti, Ioulia, 145 61 Kifissia Attica (GR)
(74) Representative: Wibbelmann, Jobst

(56) References cited:
- EP-A1- 0 427 078
- EP-A1- 1 790 356
- EP-A1- 2 674 371
- WO-A1-2007/042153
- US-A- 4 931 441

## Description

The present invention relates to a pharmaceutical composition suitable for enhancing the iron levels in blood, comprising as active ingredients iron (III) polymaltose, calcium folinate pentahydrate and vitamin C, further characterized in that the active ingredients are stored separately in a suitable device, composed of a cylindrical bottle and a closure storage cap, separated in a sealed manner. Iron (III) polymaltose is stored as an oral solution in the cylindrical bottle of the device and calcium folinate pentahydrate and vitamin C are stored in solid form in the closure storage cap of the device. The active substances which are kept in solid form in the closure storage cap are mixed with the oral solution of iron (III) polymaltose stored in the cylindrical bottle just before its use, thus resulting to a single-dose oral solution, suitable for enhancing blood iron levels.

Iron (III) polymaltose is a water-soluble complex of trivalent iron with a polysaccharide, which is useful in the treatment of iron deficiency in human body. Iron is essential in the formation of hemoglobin the major protein that is responsible for oxygen transport from the lungs to all body tissues. Iron polymaltose is considered advantageous compared to corresponding iron compositions because it is neutral and does not release free radicals being responsible for various toxic reactions in the body. In addition, iron (III) polymaltose presents high bioavailability due to its low interaction with food and other medicaments. The higher bioavailability ensures the faster synthesis of hemoglobin and myoglobin in the human body. Furthermore, iron (III) polymaltose is more stable from a chemical point of view compared to the widely used iron (II) salts which participate in oxidation reactions. Finally, iron (III) polymaltose forms stable aqueous solutions over a wide range of pH values, thus, allowing an easy handling during the preparation of pharmaceutical formulations and furthermore, simplifying its direct administration as an oral solution. This is a clear advantage especially for people who cannot consume tablets or oral solutions from effervescent tablets.

Calcium folinate pentahydrate belongs in the vitamin B family. Calcium folinate is used to prevent the side effects of certain drugs also called folic acid antagonists as them used in chemotherapy. Furthermore, calcium folinate is widely used in anemia caused during pregnancy and lactation or from liver diseases or inappropriate nutrition. Calcium folinate is fully dissolved in aqueous solutions and is absorbed in duodenum, the proximal part of the small intestine where is converted to folinic acid which is the biologically active form of folic acid. Vitamin C (ascorbic acid) presents an antioxidant activity and protects the human body from free radical formation and activity. Additionally, vitamin C contributes in the hemoglobin production and helps in iron absorption from food. Vitamin C is water soluble, but it is unstable after long storage in aqueous solutions due to oxidation reactions, while it is also sensitive to light and temperature.

EP1790356 describes compositions for use in the treatment of iron deficiencies comprising the combination of iron(III) polymaltose complex and vitamin C.

The present invention relates to a pharmaceutical composition suitable for enhancing the iron levels in blood, in the form of a single dose oral solution, which, is constituted in a single step just prior to use, from the addition of a solid preparation comprising calcium folinate pentahydrate and ervitamin C to a solution of iron (III) polymaltose. Calcium folinate pentahydrate and vitamin C are kept separately in a sealed manner, in solid form, in a closure storage cap of a system "closure storage cap-bottle", from the oral solution of iron (III) polymaltose, which is kept in the bottle, said solution is standardized in 100 mg of iron (III).

The pharmaceutical composition of the present invention comes to solve the problem of iron deficiency either in chronic patients or in temporarily iron deficient subjects for example females in pregnancy or lactation, by administrating a fortified dose of trivalent iron (100 mg per dosage unit). The pharmaceutical composition is further enriched in active substances that present a beneficial effect in iron absorption. One of these substances is calcium folinate pentahydrate, which acts as a folinic acid precursor and presents a beneficial effect in women during pregnancy and lactation. Another active substance with a similar effect is ascorbic acid with a well-established antioxidant activity and enhanced action in iron absorption.

Several containers based on the system "closure storage cap - bottle" have been reported in the state of the art, aiming to store active substances separately. In specific, one active substance in solid form is stored in the hollow space of the closure storage cap in a sealed manner from another active substance which is kept in the bottle as a solution, which said bottle is sealed with this closure storage cap. This system "closure storage cap - bottle" allows the mixture of the active substances just before their use. However, none of the prior art documents mention the use of such containers for the storage of the active ingredients and the preparation of the pharmaceutical composition of the present invention, wherein the bottle contains an iron (III) polymaltose oral solution standardized in 100 mg of trivalent iron and the closure storage cap contains an active substance in solid form that helps iron (III) absorption.

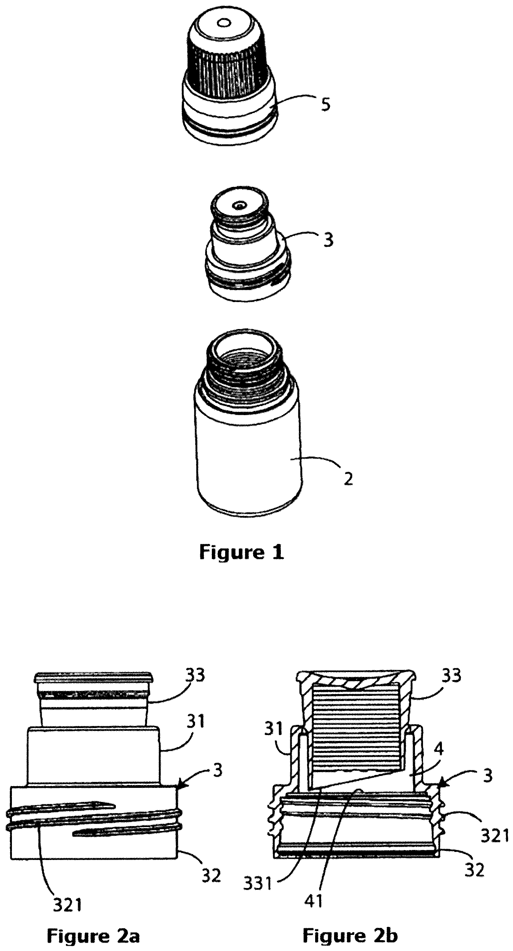

Such a suitable container based on the system "closure storage cap - bottle", is already described in details in Document EP 2 674 371 A1, herein incorporated by reference in its entirety. The features and the advantages of the system "closure storage cap - bottle", which is suitable for the present invention will be best understood upon perusal of the following detailed description, which is explanatory, with reference to the accompanying figures 1 and 2, where:
- Figure 1 illustrates an exploded view of the assembly made up of the bottle 2, the storage cap 3 and the protective closure cover 5 for the storage cap;
- Figure 2 illustrates the layout of the closure storage cap 3 that adapts to bottle 2. Figure 2a illustrates an external cross-section of the storage cap, while the Figure 2b shows an internal cross-section of the layout of the storage cap.

In this specific example, the bottle 2 has a cylindrical shape ending up to an external thread that can be screwed in the closure storage cap 3 which is threaded on the inside. The closure storage cap further comprises a protective cover 5, which is easily adjusted to the storage cap 3. Preferably, the protective cover has a shape that is similar to the shape of the device on which it is fitted.

In detail, the storage cap 3 comprises an internal cylindrical hollow space, which in combination with the outer upper portion 31, forms the internal tank 4, which is intended to house the active substance in solid form, which for the present invention will be calcium folinate pentahydrate and vitamin C to be mixed with the active substance in liquid form, namely iron (III) polymaltose standardized in 100 mg, contained in bottle 2. The base of tank 4 has a membrane, which is welded to the edge 41, and aims to the isolation of the tank 4 from the lower portion of storage cap 3, furthermore from the bottle 2, when storage cap 3 is adjusted to the mouth of bottle 2. The storage cap 3 further comprises breaking means to break the membrane at the base of tank 4, which can be manually activated by applying pressure on the top of said storage cap. Said tank breaking means comprise a cylindrical piston 33, which is able to slide inside the hollow cylindrical body of the tank 4 and is provided, on its lower portion, of at least one breaking tip 331.

The container based on the system "closure storage cap - bottle", works as described below. When the two active substances which remain separate in a sealed manner, have to be mixed, it is sufficient to remove the protective cover 5 and apply a pressure on the piston 33 of the storage cap 3. Due to this pressure, the piston 33 moves downwards until the breaking tip penetrates the membrane, thus causing the membrane to almost completely break. In this way, the first active substances in solid form contained in tank 4 fall by gravity into bottle 2, hence coming into contact with the second substance which is in liquid form. Then, the protective cover 5 can be screwed again on the storage cap 3, so as to cause the two elements (3 and 5) to be removed as one part from the mouth of the bottle 2 and allow the final user to use the content of the bottle 2.

Iron (III) polymaltose complex used in the pharmaceutical composition of the present invention is prepared following the manufacturing process of document EP 3 197 444 A1 and presents the technical features of Table 1.

**Table 1. Technical parameters and specification limits of Iron (III) polymaltose complex.**

| **Technical Parameters** | **Specifications** |
|---|---|
| Color | Brown - Red |
| Solubility | Soluble in water |
| Loss on Drying | < 8 % w/w |
| pH 5% w/v in Fe³⁺ solution | 5.5 - 7.5 |
| Free Iron in 5% w/v σε Fe³⁺ solution | < 0.05 % w/w |
| Complexed Fe³⁺ on dry base | 28.0 - 36.0 % w/w |
| Total Polymaltose on dry base | 25.0 - 50 % w/w |
| Free Chlorides (as NaCl) on dry base | < 3 % |
| TAMC | < 1000 cfu/g |
| TYMC | < 100 cfu/g |
| E. coli | Absence in 1.0 gr |

In a preferred embodiment, the quantity of iron (III) polymaltose complex in the pharmaceutical composition of the present invention is in the range from 285 mg to 360 mg per dosage unit and it is adjusted accordingly in order to correspond to 100 mg of trivalent iron per dosage unit of the pharmaceutical composition. The solution of iron (III) polymaltose complex further comprises pharmaceutically acceptable excipients, including, solvents, sweeteners, flavorings and preservatives.

In a preferred embodiment, each bottle of the container as described in the present invention contains 5 mL of an oral solution of iron (III) polymaltose complex, which corresponds to 100 mg trivalent iron, while, the closure storage cap contains 200 mg of a solid preparation based on calcium folinate pentahydrate.

In a preferred embodiment, each bottle of the container as described in the present invention contains 5 mL of an oral solution of iron (III) polymaltose complex, which corresponds to 100 mg trivalent iron, while, the closure storage cap contains 500 mg of vitamin C.

In a preferred embodiment, each bottle of the container as described in the present invention contains 5 mL of an oral solution of iron (III) polymaltose complex, which corresponds to 100 mg trivalent iron, while, the closure storage cap contains 500 mg of vitamin C and 200 mg of a solid preparation based on calcium folinate pentahydrate.

The invention is further described in the following examples.

Example 1: Preparation and conservation of solid active substance contained in the closure storage cap.

The solid mixture comprising calcium folinate pentahydrate and mannitol is homogenized in a drum blender for 30 minutes at 45 rpm. Uniformity analysis performed to verify homogenization and the final blend placed in the closure storage caps and sealed air tight.

The solid pharmaceutical preparation in the storage cap as described in the present invention has the following composition:

| **Ingredients in solid form (total quantity 200 mg)** | **Quantity (mg)** |
|---|---|
| Calcium Folinate Pentahydrate | 0.235 mg (corresponds to 0.185 mg folinic acid) |
| Mannitol powder | Up to 200 mg |

Example 2: Preparation and conservation of the liquid active substance contained in the bottle.

In a stainless steel reactor, a quantity of purified water, corresponding to half of the total quantity used in the formulation, was placed and heated at 70 ± 5 °C under stirring. Then, Iron (III) polymaltose complex was added in portions at the same temperature under stirring. After iron (III) polymaltose complex was fully dissolved, the addition of sucrose and sorbitol was followed and the new mixture was stirred at the same temperature until it is fully homogenized. Subsequently, the solution cooled to 35 ± 5 °C, following the addition of preservatives (p-hydroxybenzoic methylester and p-hydroxybenzoic propylester) and the flavoring agent. Then, the solution cooled to room temperature and pH is adjusted in the range between 5.5 and 7.0 in the aid of NaOH 1N or HCl 1N. Finally, purified water was added up to the final volume and the solution was filtered through membrane filter of 150 µm to ensure the removal of any undissolved matter. The filtered brown-red solution is bottled in the container which subsequently sealed with the closure storage cap.

The pharmaceutical preparation in liquid form contained in the bottle has the following composition:

| **Ingredients in liquid form (Final Volume 5 mL)** | **Quantity (mg)** |
|---|---|
| Iron (III) polymaltose complex | 300* (corresponds to 100 mg Fe³⁺) |
| Sorbitol 70% | 1000 |
| Sucrose | 500 |
| *p*-hydroxybenzoic methylester | 9 |
| *p*-hydroxybenzoic propylester | 1 |
| Flavoring | 3 |
| Purified Water | qs 5 mL |

| | |
|---|---|
| ^{∗} The quantity of iron (III) polymaltose complex in the composition of the invention is adjusted in such a way so that the amount of trivalent iron in the final solution is 100 mg | |

### Literature

EP 3 197 444 A1 Iron (III) hydroxide complexes with activated glucose syrups and process for preparing same.
EP 2 674 371 A1 Bottle cap for the conservation of substances to be kept separate until their use.

## Claims

1. A pharmaceutical composition for use as a single dose oral solution comprising iron (III) polymaltose complex, calcium folinate and vitamin C, wherein the iron (III) polymaltose complex is stored as a solution in a cylindrical bottle and calcium folinate and vitamin C are stored separate from said solution, in solid form, in a storage space in the closure cap of the bottle.

2. The pharmaceutical composition according to claim 1 wherein the iron (III) polymaltose complex is standardized in 32 ± 4% w/w in trivalent iron.

3. The pharmaceutical composition according to claims 1 and 2 wherein the quantity of trivalent iron is 100 mg per dosage unit of oral solution.

## Patentansprüche

1. Pharmazeutische Zusammensetzung zur Verwendung als orale Einzeldosislösung, umfassend Eisen(III)-Polymaltosekomplex, Calciumfolinat und Vitamin C, wobei der Eisen(III)-Polymaltosekomplex als Lösung in einer zylindrischen Flasche gelagert wird und Calciumfolinat und Vitamin C getrennt von der besagten Lösung in fester Form in einem Lagerraum in der Verschlusskappe der Flasche gelagert werden.

2. Die pharmazeutische Zusammensetzung nach Anspruch 1, wobei der Eisen(III)-Polymaltosekomplex auf 32 ± 4 Gew.-% dreiwertiges Eisen standardisiert ist.

3. Die pharmazeutische Zusammensetzung nach den Ansprüchen 1 und 2, wobei die Menge an dreiwertigem Eisen 100 mg pro Dosierungseinheit der oralen Lösung beträgt.

## Revendications

1. Composition pharmaceutique pour une utilisation comme solution buvable monodose comprenant un complexe fer (III) polymaltose, du folinate de calcium et de la vitamine C, dans laquelle le complexe fer (III) polymaltose est stocké sous la forme d'une solution dans un flacon cylindrique et le folinate de calcium et la vitamine C sont stockés séparément de ladite solution, sous forme solide, dans un espace de stockage du bouchon de fermeture du flacon.

2. Composition pharmaceutique selon la revendication 1, dans laquelle le complexe fer (III) polymaltose est standardisé à 32 ± 4 % p/p en fer trivalent.

3. Composition pharmaceutique selon les revendications 1 et 2, dans laquelle la quantité de fer trivalent est de 100 mg par dose unitaire de solution buvable.
